Europäisches Patentamt

European Patent Office

Office européen des brevets

(19)

(11) Publication number: **0 349 037**
**A1**

(12) **EUROPEAN PATENT APPLICATION**

(21) Application number: **89201560.3**

(22) Date of filing: **15.06.89**

(51) Int. Cl.⁴: **G01N 33/543 , G01N 33/569 , //G01N33/76**

(30) Priority: **27.06.88 NL 8801626**

(43) Date of publication of application:
**03.01.90 Bulletin 90/01**

(84) Designated Contracting States:
**AT BE CH DE ES FR GB GR IT LI NL SE**

(71) Applicant: **AKZO N.V.**
**Velperweg 76**
**NL-6824 BM Arnhem(NL)**

(72) Inventor: **Hellings, Jan Albert, Dr.**
**Thorbeckestraat 20**
**NL-5344 SN Oss(NL)**

(74) Representative: **Hermans, Franciscus G.M. et al**
**Patent Department AKZO N.V. Pharma**
**Division P.O. Box 20**
**NL-5340 BH Oss(NL)**

(54) **Method for detection of antibodies or antigens in a test fluid.**

(57) The invention concerns a method for detection of one or more antibodies or antigens in a test fluid which antibodies or antigens each possess at least a corresponding recognition site available for a binding reaction. The test fluid together with a labelled component is brought into contact with an insoluble component. Each of the components consist of one or more antigens or antibodies whereby the antibodies of one component may not be specifically directed against the antigen of the other component. After the reaction the insoluble phase is separated from the liquid phase and the determination of the labelling compound indicates whether the antibodies or antigens to be detected are present in the test fluid.

EP 0 349 037 A1

## Method for detection of antibodies or antigens in a test fluid.

The invention relates to a method for the detection of one or more antibodies or antigens in a test fluid, which antibodies or antigens each possess at least a corresponding recognition site available for a binding reaction, and also a test kit to be used for the said detection.

A number of problems arise when carrying out a method of this type. If the test fluid contains one or more antibodies or antigens which give cross-reactions with one and the same immunocomponent, the weakly cross-reacting antibodies or antigens will not be shown in the test fluid because these antibodies or antigens are not able to compete sufficiently with specific other antibodies or antigens which are present in the test fluid.

In this way cross-reacting antibodies will not be indicated in an inhibition assay because these are less well able to displace a labelled specific antibody from the specific antigen on the solid phase. These problems arise with those tests in which the test fluid contains cross-reacting antibodies or antigens. Examples of such tests are those in which gonadotrophic substances, such as hCG (human chorionic gonadotrophin), LH (luteinizing hormone) and FSH (follicle-stimulating hormone), have to be measured. This problem also arises in the detection of antibodies directed against human immunodeficiency virus (anti-HIV) in human serum or plasma.

To enable each of the various cross-reacting antibodies or antigens in a test fluid to be detected individually it is necessary to carry out a number of detections for this purpose. Thus for the detection of anti-HIV-1 using an inhibition assay it is necessary to use a HIV-1 antigen and a labelled specific anti-HIV-1 as conjugate.

If the test fluid, for example serum, contains anti-HIV-1, this antibody will compete with the labelled human anti-HIV-1 for the available binding sites on the HIV-1 antigen. If the serum contains antibodies directed against other human immunodeficiency virus strains, such as HIV-2, these HIV-2 antibodies will not be shown because these are not able to inhibit the binding of the more strongly binding HIV-1 antibodies of the conjugate.

Consequently a large number of tests must be carried out to obtain insight into whether a test fluid contains an antigen or an antibody and which antibody is present. Frequently, routine laboratories, such as blood banks, are interested only in whether a blood donor is carrier of a virus causing AIDS. As it is to be expected that between now and the near future various virus strains which cause AIDS will be discovered, a blood bank will therefore have to test the blood, or a derived fraction thereof, for the various viruses. Time-consuming, expensive and numerous tests will have to be carried out for this to be able to come to an unambiguous conclusion.

It is characteristic for the invention that the test fluid, together with a labelled component, is brought into contact with a component which has been rendered insoluble, each of the components consisting of one or more antigens or antibodies which can occur in the test fluid or which are directed against one or more antigens or antibodies in the test fluid, whereby the antibody or antibodies of one component may not be specifically directed against the antigen or antigens of the other component, after which the insoluble phase is separated from the liquid phase and the determination of the labelling compound in one or both phases indicates whether the antibodies or antigens to be detected are present. The invention also relates to a test kit to be used for the abovementioned detection. The test kit contains at least a component which has been rendered insoluble and a labelled component, each of the components consisting of one or more antibodies or antigens and the antibody or antibodies of one component not being specifically directed against the antigen or antigens of the other component.

The method for the detection of one or more antibodies or antigens in a test fluid is preferably used for the detection of one or more antibodies directed against various HIV strains. The component which has been rendered insoluble being provided with at least one of the human immunodeficiency viruses as antigen and a labelled antibody being used as the labelled component, which antibody is directed against a different human immunodeficiency virus than that which is used as the antigen rendered insoluble.

A test kit to be used for this said detection must contain a component which has been rendered insoluble and which is provided with one of the human immunodeficiency viruses as antigen and a labelled antibody, which antibody is directed against a different human immunodeficiency virus than that which is used as the antigen rendered insoluble.

In particular, the method according to the invention is used for the detection of one or more antibodies in a test fluid, which antibodies are directed against different HIV strains, such as HIV-1 and HIV-2. A HIV-2 antigen is used as coating on the solid phase and whereby the labelled antibody conjugate is directed against HIV-1.

A test kit to be used for this lastmentioned detection contains a human immunodeficiency virus type 2 antigen, which has been rendered insoluble, and a labelled antibody directed against human immunodeficiency virus type 1.

The method according to the invention for the detection of one or more antibodies or antigens in a test fluid, specifically the detection of HIV antibodies in serum or plasma, will be explained in more detail.

It is known that AIDS (Acquired Immunodeficiency Syndrome) and ARC (AIDS-Related Complex) are caused by a retrovirus and that many variants of this virus exist. Various names have been used for this virus in the past, such as HTLV-III (Human T Lymphotropic Virus type III), LAV (Lymphadenopathy-Associated Virus) and ARV (AIDS-associated RetroVirus). At present, HIV (Human Immunodeficiency Virus) is generally accepted as the designation of the retrovirus which causes AIDS. At present two groups of this HIV are known, i.e. HIV-1 and HIV-2. Depending on the virus with which an individual is infected, HIV-1 or HIV-2, HIV-1 antibodies or HIV-2 antibodies are evoked.

For the detection of HIV-1 antibodies, a HIV-1 antigen is frequently used on the solid phase and anti-HIV-1 is used as conjugate. If, for example, the test fluid now contains a small amount of antibodies directed against HIV-2, these antibodies will not be detected because the more strongly binding anti-HIV-1 as conjugate will suppress the HIV-2 antibodies in the competition for a binding position on the HIV-1 antigen. By applying a HIV-2 antigen in place of a HIV-1 antigen to the solid phase, both HIV-1 and HIV-2 antibodies occurring in the serum will be detected, while a labelled HIV-1 antibody has been used as conjugate. By this means a method is obtained with which both antibodies, anti-HIV-1 and anti-HIV-2, are detected in the serum. A great advantage of this method is that the presence of said HIV antibodies to be detected, is performed in one test. Another embodiment with which both antibodies are likewise detected is that HIV-1 antigen is coated on the solid phase and a labelled HIV-2 antibody is used as conjugate.

It is also possible according to this invention to detect in serum three antibodies, for example HIV-1 antibody, HIV-2 antibody and a fictive HIV-3 antibody, by binding HIV-1 antigen and HIV-2 antigen on the solid phase and using a labelled fictive HIV-3 antibody as conjugate.

A special embodiment with which two antibodies can likewise be detected in the serum is that in which, a HIV-1 antibody is coated on the solid phase and a labelled HIV-2 antigen is used as conjugate.

With the detection method according to the invention, the test fluid, together with a labelled component, is brought into contact with a component which has been rendered insoluble.

"A component which has been rendered insoluble" means any component which is bonded to carrier material. Carrier materials which can be used are, for example, test tubes, microtitration plates, small spheres, discs or rods made of, for example, plastic or glass.

A "labelled component" means a component provided with a labelling compound to be determined directly or indirectly. Labelling compounds which can be used are enzymes, radioactive substances, gold sols, dyestuff sols or fluorescent compounds. Preferably an enzyme is used as the labelling compound. Determination of this labelling compound is subsequently effected by using a substrate solution suitable for the enzyme, by which means the colour of the solution can change, which gives a measure for the presence of the antibodies or antigens to be detected.

The invention is illustrated with the aid of the following example:

## Example

Polystyrene microtitration plates (8x12 incubation wells) were incubated for 16 hours at 4°C with HIV-2 virus lysate obtained from the supernatant liquor from a culture of a human T cell line infected with the virus. The viral lysate was diluted to a concentration of 2 micrograms per litre 0.05 M $NaHCO_3$, pH 9.6. After drawing off the coat fluid, the wells were rinsed with a solution of 0.05 M phosphate-buffered saline solution pH 7.4 to which 4% (w/w) bovine serum albumin had been added. After removing the rinse liquid, the plates were dried at a relative humidity of 15% at room temperature and stored in an airtight container with silica gel as the dessicant. Detection of anti-HIV-1 and anti-HIV-2 was effected by incubating sera (100 microlitres) in the wells coated with HIV-2 antigen for 90 minutes at 37°C together with anti-HIV-1 immunoglobulin to which the enzyme HRP (Horse Radish Peroxidase) was coupled. The concentration of the anti-HIV-1-HRP conjugate was so chosen that an extinction value at 492 mm of 1.0 to 1.5 A was obtained when anti-HIV-1 (or -2) negative serum was used as the test fluid. After incubation, the fluid was drawn off from the wells and the wells were washed with 0.05 M PBS +0.01% Tween-20(R). The substrate (100 microlitres) for the enzyme detection was orthophenylenediamine (OPD) with urea peroxide. After 30 minutes at room temperature the reaction was stopped by adding 100 μl 1 mol/litre $H_2SO_4$. The extinction

at 492 nm was determined in a colorimeter.

For comparison, the same test was carried out in incubation wells which were coated with HIV-1 antigen.

The results are summarized in Table 1.

Table 1

| Coat antigen | Test Fluid | E 492 nm | Result |
|---|---|---|---|
| HIV-1 | anti-HIV-1 serum (human) | 0.14 | + |
| HIV-1 | anti-HIV-2 serum (human) | 0.92 | - |
| HIV-1 | negative control serum (human) | 1.20 | - |
| HIV-2 | anti-HIV-1 serum (human) | 0.36 | + |
| HIV-2 | anti-HIV-2 serum (human) | 0.21 | + |
| HIV-2 | negative control serum (human) | 1.16 | + |

**Claims**

1. Method for the detection of one or more antibodies or antigens in a test fluid, which antibodies or antigens each possess at least a corresponding recognition site available for a binding reaction, characterized in that the test fluid, together with a labelled component, is brought into contact with a component which has been rendered insoluble, each of the components consisting of one or more antigens or antibodies which can occur in the test fluid or which are directed against one or more antigens or antibodies in the test fluid, whereby the antibody or antibodies of one component may not be specifically directed against the antigen or antigens of the other component, after which the insoluble phase is separated from the liquid phase and the determination of the labelling compound in one or both phases indicates whether the antibodies or antigens to be detected are present.

2. Method for the detection of one or more antibodies or antigens in a test fluid according to Claim 1, characterized in that the component which has been rendered insoluble is provided with at least one of the human immunodeficiency viruses as antigen and a labelled antibody is used as the labelled component, which antibody is directed against a different human immunodeficiency virus than that which is used as the antigen rendered insoluble.

3. Method for the detection of one or more antibodies or antigens in a test fluid according to Claim 2, characterized in that a human immunodeficiency virus type 2 is used as the antigen rendered insoluble and the labelled antibody is directed against human immunodeficiency virus type 1.

4. Method for the detection of one or more antibodies or antigens in a test fluid according to Claims 1-3, characterized in that an enzyme is used as the labelling compound.

5. Test kit to be used for the detection according to Claim 1, wherein the test kit contains a component which has been rendered insoluble and a labelled component, each of the components consisting of one or more antibodies or antigens and the antibody or antibodies of one component not being specifically directed against the antigen or antigens of the other component.

6. Test kit to be used for the detection according to Claim 2, wherein an insoluble component provided with at least one of the human immunodeficiency viruses as antigen and a labelled antibody, which antibody is directed against a different human immunodeficiency than that which is used as the antigen rendered insoluble, are present.

7. Test kit to be used for the detection according to Claim 3, wherein at least a human immunodeficiency virus type 2 antigen, which has been rendered insoluble, and a labelled antibody directed against human immunodeficiency virus type 1 are present.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | JOURNAL OF CLINICAL MICROBIOLOGY, vol. 26, no. 5, May 1988, pages 1000-1004, American Society for Microbiology; F. DENIS et al.: "Comparison of 10 enzyme immunoassays for detection of antibody to human immunodeficiency virus type 2 in West African Sera" * Whole document * | 1-7 | G 01 N 33/543 G 01 N 33/569// G 01 N 33/76 |
| A | BRITISH MEDICAL JOURNAL, vol. 296, 9th January 1988, pages 83-86; D.C.W. MABEY et al.: "Human retroviral infections in The Gambia: prevalence and clinical features" * Whole article * | 1-7 | |
| A | EP-A-0 260 599 (DIAGEN) * Pages 1-5 * | 1-7 | |
| A | EP-A-0 135 378 (DAINIPPON PHARMACEUTICAL CO., LTD) * Abstract; pages 1-7 * | 1,4,5 | TECHNICAL FIELDS SEARCHED (Int. Cl.5) |
| A | THE LANCET, vol. I, no. 8525, 17th January 1987, pages 128-132; F. BRUN-VEZINET et al.: "Lymphadenopathy-associated virus type 2 in AIDS and AIDS-related complex" * Abstract; page 129, column 2; page 131, column 2; page 132, column 1 * | 1-7 | G 01 N |

-/-

The present search report has been drawn up for all claims

| Place of search | Date of completion of the search | Examiner |
|---|---|---|
| THE HAGUE | 04-09-1989 | HITCHEN C.E. |

**European Patent Office**

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,P | THE LANCET, vol. II, no. 8617, 22nd October 1988, pages 927-930, The Lancet Ltd, London, GB; R.S. TEDDER et al.: "Envelope cross-reactivity in western blot for HIV-1 and HIV-2 may not indicate dual infection" * Whole article * | 1-7 | |

TECHNICAL FIELDS SEARCHED (Int. Cl.5)

The present search report has been drawn up for all claims

| Place of search | Date of completion of the search | Examiner |
|---|---|---|
| THE HAGUE | 04-09-1989 | HITCHEN C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P0401)